(19) Europäisches Patentamt
European Patent Office
Office européen des brevets

(11) Veröffentlichungsnummer: **0 056 124**
**B1**

(12) # EUROPÄISCHE PATENTSCHRIFT

(45) Veröffentlichungstag der Patentschrift: **27.06.84**

(51) Int. Cl.³: **C 07 C 127/22, A 01 N 47/34**

(21) Anmeldenummer: **81110349.8**

(22) Anmeldetag: **11.12.81**

(54) **N-Benzoyl-N'-phenylharnstoffe, Verfahren zu ihrer Herstellung und ihre Verwendung zur Bekämpfung von Insekten.**

(30) Priorität: **14.01.81 DE 3100911**

(43) Veröffentlichungstag der Anmeldung: **21.07.82 Patentblatt 82/29**

(45) Bekanntmachung des Hinweises auf die Patenterteilung: **27.06.84 Patentblatt 84/26**

(84) Benannte Vertragsstaaten: **AT BE CH DE FR GB IT LI LU NL SE**

(56) Entgegenhaltungen:
**EP - A - 0 009 762**

**JOURNAL OF AGRICULTURAL AND FOOD CHEMISTRY, Band 21, Nr. 3, Mai/Juni 1973, Seiten 348-354, American Chemical Society N.W. Washington D.C. (USA); K.WELLINGA et al.: "Synthesis and laboratory evaluation of 1-(2,6-Disubstituted benzoyl)-3-phenylureas, a new class of insecticides. I. 1-(2,6-Dichlorobenzoyl)-3-phenylureas"**

(73) Patentinhaber: **BASF Aktiengesellschaft, Carl-Bosch-Strasse 38, D-6700 Ludwigshafen (DE)**

(72) Erfinder: **Lange, Arno, Dr., Friedrichsplatz 11, D-6800 Mannheim (DE)**
Erfinder: **Kiehs, Karl, Dr., Sudetenstrasse 22, D-6840 Lampertheim (DE)**
Erfinder: **Adolphi, Heinrich, Dr., Kalmitweg 11, D-6703 Limburgerhof (DE)**

**EP 0 056 124 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft N-Benzoyl-N'-phenylharnstoffe, Verfahren zu ihrer Herstellung und insektizide Mittel, die diese Verbindungen als Wirkstoffe enthalten.

Es ist bekannt, daß N-Benzoyl-N'-phenylharnstoffe insektizid wirksam sind (DE-OS 2 843 851).

Es wurde gefunden, daß N-Benzoyl-N'-phenylharnstoffe der Formel

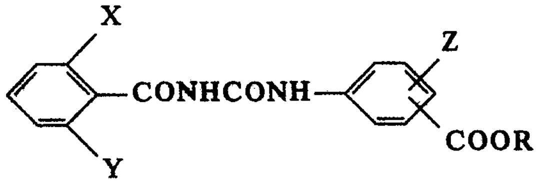

(I)

in der

X für Chlor, Fluor oder Methyl,
Y für Chlor, Fluor oder Wasserstoff,
Z für Brom, Chlor, Fluor oder Wasserstoff und
R für einen primären oder sekundären Alkylrest mit 3 bis 12 Kohlenstoffatomen oder einen gegebenenfalls durch Alkyl substituierten Cycloalkylrest mit 5 bis 12 Kohlenstoffatomen oder den Adamantanylrest stehen,

insektizid sehr wirksam sind. Sie sind bekannten Benzoylharnstoffen ähnlicher Struktur in ihrer Wirkung überlegen.

Als Alkylreste für R in Formel I kommen primäre und sekundäre Alkylreste mit 3 bis 12 Kohlenstoffatomen, wie Isopropyl, sec-Butyl, Pentyl-(3), 2,4-Dimethyl-pentyl-(3), n-Heptyl, 2,6-Dimethyl-heptyl-(4), 2,2,4,4-Tetramethyl-pentyl-(3), 1-Methyl-7-ethyl-nonyl-(4), 2,8 Dimethyl-nonyl-(8), 3,5,5-Trimethylhexyl-(1), in Betracht. Geeignete Cycloalkylreste mit 5 bis 12 Kohlenstoffatomen, die durch Alkyl, insbesondere mit 1 bis 4 Kohlenstoffatomen, substituiert sein können, sind beispielsweise Cyclopentyl, Cyclohexyl oder 4-tert.-Butyl-cyclohexyl.

Bevorzugte N-Benzoyl-N'-phenylharnstoffe sind Verbindungen der Formel I, in der X Fluor oder Chlor und Y Fluor oder Wasserstoff bedeuten und der Rest —COOR sich in p-Stellung befindet. R ist vorzugsweise ein sekundärer Alkylrest mit 4 bis 9 Kohlenstoffatomen.

Man erhält die N-Benzoyl-N'-phenylharnstoffe der Formel I durch Umsetzung von Benzoylisocyanaten der Formel

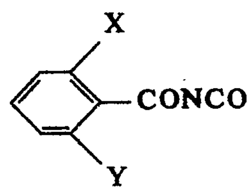

(II)

in der X und Y die obengenannten Bedeutungen haben, mit Aminobenzoesäureestern der Formel

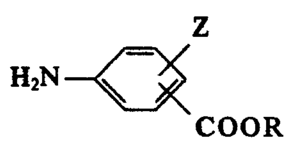

(III)

in der Z und R die obengenannte Bedeutungen haben, in Gegenwart eines inerten organischen Lösungsmittels bei einer Temperatur im Bereich zwischen 0 und 80° C.

Das Verfahren zur Herstellung der erfindungsgemäßen Verbindungen wird in Gegenwart geeigneter Lösungs- und Verdünnungsmittel durchgeführt. Als solche kommen praktisch alle inerten organischen Solventien in Betracht. Hierzu gehören insbesondere aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan; cyclische und acyclische Ether, wie Diethylether, Dibutylether, Tetrahydrofuran, Dioxan; acyclische und cyclische Ketone, wie Aceton, Methylethylketon, Methylisopropylketon, Cyclohexanon; Nitrile, wie Acetonitril, Benzonitril. Auch Gemische dieser Lösungsmittel können verwendet werden.

Die Reaktionstemperatur kann innerhalb eines größeren Bereichs variiert werden. Sie liegt in der Regel zwischen 0 und 80° C. Die Umsetzung verläuft aufgrund der exothermen Reaktion üblicherweise bei einer Temperatur im Bereich zwischen 20 und 60° C.

Man läßt die Umsetzung im allgemeinen bei Normaldruck ablaufen. Sie kann diskontinuierlich oder

kontinuierlich durchgeführt werden.

Zur Durchführung der Verfahren setzt man die Reaktionskomponenten vorzugsweise in äquimolarem Verhältnis ein. Ein Überschuß der einen oder anderen Komponente bringt keine wesentlichen Vorteile. Die Umsetzung verläuft praktisch quantitativ.

Die Harnstoffe der Formel I fallen als Festprodukte an, die in der Regel analysenrein sind, andernfalls aber durch Umkristallisieren gereinigt werden können. Zu ihrer Charakterisierung dienen Elementaranalyse und Schmelzpunkt.

Zweckmäßigerweise wird der substituierte Aminobenzoesäureester der Formel III zusammen mit dem Lösungs- oder Verdünnungsmittel vorgelegt, dann wird das Isocyanat der Formel III zugegeben. Nach mehrstündiger Reaktionsdauer, in der Regel 2 Stunden, wird das Produkt dann abgesaugt und unter vermindertem Druck getrocknet.

Methoden der Herstellung für die Benzoylisocyanate der Formel II und die Aminobenzoesäureester der Formel III sind bekannt (J. Org. Chem. 28, 1805—1811 (1963); Organicum, 9. Auflage, 1969, S. 446, 576, VEB Deutscher Verlag der Wissenschaften).

Die Harnstoffe der Formel I können auch durch Umsetzung von Amiden der Formel

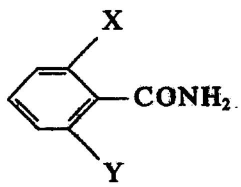

(IV)

mit Isocyanaten der Formel

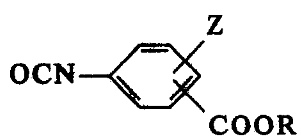

(V)

hergestellt werden. In den Formeln IV und V haben X, Y, Z und R die obengenannten Bedeutungen.

Die Reaktionspartner werden dabei in äquimolaren Mengen gegebenenfalls in Gegenwart eines inerten organischen Lösungsmittels umgesetzt. Die Reaktionstemperatur liegt zwischen 0 und 140°C, vorzugsweise zwischen 60 und 100°C. Gegebenenfalls kann ein Katalysator, wie Triethylamin, zugesetzt werden. Als Lösungsmittel kommen die gleichen Solventien in Betracht, die bei der Umsetzung der Verbindungen der Formel II mit den Verbindungen der Formel III verwendet werden können.

## Herstellungsbeispiel

6,75 g 4-Amino-2-chlorbenzoesäure-(2,4-dimethylpentyl-3)-ester werden in 80 ml absolutem Toluol gelöst und tropfenweise mit 4,6 g 2,6-Difluorbenzoylisocyanat versetzt. Dann hält man für 2 Stunden bei 50°C und saugt bei 0°C ab. Anschließend wird im Trockenschrank unter vermindertem Druck bei 40°C getrocknet. Man erhält 8,1 g N-(2,6-Difluorbenzoyl)-N'-[3-chlor-4-(2,4-dimethyl-pentoxy-3-carbonyl)-phenyl]-harnstoff (Verbindung Nr. 1)
Fp.: 135—140°C.

Folgende Verbindungen lassen sich beispielsweise nach einem der oben beschriebenen Verfahren herstellen:

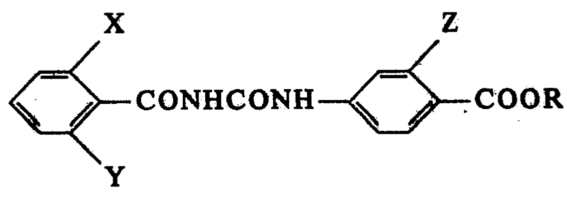

| Nr. | X | Y | Z | R | Fp [°C] |
|---|---|---|---|---|---|
| 2 | Cl | H | Cl | 2,4-Dimethyl-pentyl-(3) | 152—154 |
| 3 | F | F | Cl | 3,5,5-Trimethyl-n-hexyl | 114—115 |
| 4 | Cl | Cl | Cl | 3,5,5-Trimethyl-n-hexyl | 185—186 |
| 5 | Cl | H | Cl | 3,5,5-Trimethyl-n-hexyl | 125—126 |

| Nr. | X | Y | Z | R | Fp [°C] |
|---|---|---|---|---|---|
| 6 | $CH_3$ | H | Cl | 3,5,5-Trimethyl-n-hexyl | 148—150 |
| 7 | F | F | Cl | n-Heptyl | 100—108 |
| 8 | Cl | Cl | Cl | n-Heptyl | 125—127 |
| 9 | Cl | H | Cl | n-Heptyl | 126—128 |
| 10 | $CH_3$ | H | Cl | n-Heptyl | 133—135 |
| 11 | F | F | Cl | 2,6-Dimethyl-heptyl-(4) | 140—142 |
| 12 | Cl | Cl | Cl | 2,6-Dimethyl-heptyl-(4) | 145—147 |
| 13 | Cl | H | Cl | 2,6-Dimethyl-heptyl-(4) | 144—146 |
| 14 | $CH_3$ | H | Cl | 2,6-Dimethyl-heptyl-(4) | 104—106 |
| 15 | F | F | Cl | Pentyl-(3) | 150—152 |
| 16 | Cl | Cl | Cl | Pentyl-(3) | 160—165 |
| 17 | Cl | H | Cl | Pentyl-(3) | 149—150 |
| 18 | F | F | Cl | 1-Methyl-7-ethyl-nonyl-(4) | 97— 99 |
| 19 | Cl | Cl | Cl | 1-Methyl-7-ethyl-nonyl-(4) | Öl ($n_D^{25}$ =1.5541) |
| 20 | Cl | H | Cl | 1-Methyl-7-ethyl-nonyl-(4) | 90— 95 |
| 21 | $CH_3$ | H | Cl | 1-Methyl-7-ethyl-nonyl-(4) | 50— 53 |
| 22 | F | F | Cl | Adamantanyl | 237—242 |
| 23 | Cl | Cl | Cl | Adamantanyl | 232—234 |
| 24 | Cl | H | Cl | Adamantanyl | 234—240 |
| 25 | F | F | Br | 2,4-Dimethyl-pentyl-(3) | 149—151 |
| 26 | F | F | H | 2,6-Dimethyl-heptyl-(4) | 154—156 |
| 27 | Cl | Cl | H | 2,6-Dimethyl-heptyl-(4) | 167—169 |
| 28 | F | F | Cl | 4-tert.-Butyl-cyclohexyl | 217—220 |
| 29 | F | H | Cl | 4-tert.-Butyl-cyclohexyl | 217—220 |
| 30 | Cl | H | H | 2,6-Dimethyl-heptyl-(4) | |
| 31 | F | F | H | 2,4-Dimethyl-pentyl-(3) | 155—159 |
| 32 | F | Cl | H | 2,4-Dimethyl-pentyl-(3) | |
| 33 | Cl | Cl | H | 2,4-Dimethyl-pentyl-(3) | 172—177 |
| 34 | Cl | H | H | 2,4-Dimethyl-pentyl-(3) | 163—165 |
| 35 | F | F | Cl | 2,2,4,4-Tetramethylpentyl-(3) | |

| Nr. | X | Y | Z | R | Fp [°C] |
|---|---|---|---|---|---|
| 36 | Cl | Cl | Cl | 2,2,4,4-Tetramethylpentyl-(3) | |
| 37 | Cl | H | Cl | 2,2,4,4-Tetramethylpentyl-(3) | |
| 38 | F | F | F | 2,4-Dimethyl-pentyl-(3) | |
| 39 | Cl | Cl | F | 2,4-Dimethyl-pentyl-(3) | |
| 40 | Cl | H | F | 2,4-Dimethyl-pentyl-(3) | |
| 41 | F | F | F | 2,6-Dimethyl-heptyl-(4) | |
| 42 | Cl | Cl | F | 2,6-Dimethyl-heptyl-(4) | |
| 43 | Cl | H | F | 2,6-Dimethyl-heptyl-(4) | |
| 44 | F | F | Cl | sec.-Butyl | 168—172 |
| 45 | Cl | Cl | Cl | sec.-Butyl | 165—169 |
| 46 | Cl | H | Cl | sec.-Butyl | 155—157 |
| 47 | F | F | Cl | Isopropyl | |
| 48 | Cl | Cl | Cl | Isopropyl | |
| 49 | Cl | H | Cl | Isopropyl | |
| 50 | F | F | F | Pentyl-(3) | |
| 51 | Cl | Cl | F | Pentyl-(3) | |
| 52 | Cl | H | F | Pentyl-(3) | |

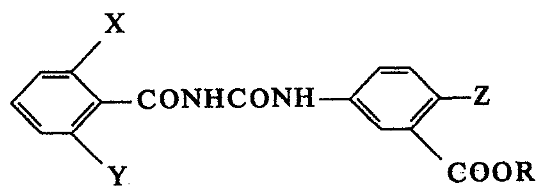

| Nr. | X | Y | Z | R | Fp [°C] |
|---|---|---|---|---|---|
| 53 | F | F | Cl | 2,4-Dimethyl-pentyl-(3) | 172—174 |
| 54 | Cl | Cl | Cl | 2,4-Dimethyl-pentyl-(3) | 155—158 |
| 55 | Cl | H | Cl | 2,4-Dimethyl-pentyl-(3) | 195—198 |
| 56 | F | F | Cl | 2,6-Dimethyl-n-heptyl-(4) | 180—182 |
| 57 | Cl | Cl | Cl | 2,6-Dimethyl-n-heptyl-(4) | 178—181 |
| 58 | Cl | H | Cl | 2,6-Dimethyl-n-heptyl-(4) | 146—150 |
| 59 | F | F | Cl | 2,8-Dimethyl-nonyl-(5) | 130—134 |
| 60 | Cl | Cl | Cl | 2,8-Dimethyl-nonyl-(5) | 100—104 |
| 61 | Cl | H | Cl | 2,8-Dimethyl-nonyl-(5) | 110—112 |

Die erfindungsgemäßen N-Benzoyl-N'-phenylharnstoffe der Formel I sind geeignet, Schädlinge aus der Klasse der Insekten wirksam zu bekämpfen. Sie können im Pflanzenschutz sowie auf dem Hygiene-, Vorratsschutz- und Veterinärsektor als Schädlingsbekämpfungsmittel eingesetzt werden.

Zu diesen schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pinno (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarus (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus Pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor (Hessenfliege), Dasineura brassicae (Kohlschoten-Gallmücke), Contarinie tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschenfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ocis, Chrysomya macellaria, Chrysomya hominivorax, Lucilia cuprina, Lucilia sericata, Hypoderma lineata;

aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);

aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);

aus der Ordnung der Pflanzensauger (Homoptera) beispielsweise Perkiniella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysphis radicola (Mehlige Apfelfalterlaus), Brachycaudus cardui (Große Pflaumenblattlaus), Brevicornye brassicae (Kohlblattlaus), Phorodon humuli (Hopfenblattlaus), Rhopalomyzus ascalonicus (Zwiebellaus), Myzodes persicae (Grüne Pfirsichlaus), Myus

cerasi (Schwarze Sauerkirschenlaus), Dysaulacorthum pseudosolani (Gefleckte Kartoffellaus), Acyrthosiphon onobrychis (Grüne Erbsenlaus), Macrosiphon rasae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifugus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronapthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, Gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Beispiele für Formulierungen sind:

I. 5 Gewichtsteile der Verbindung Nr. 14 werden mit 95 Gewichtsteilen feinteiligem Kaolin innig vermischt. Man erhält auf diese Weise ein Stäubemittel, das 5 Gew.-% des Wirkstoffs enthält.

II. 30 Gewichtsteile der Verbindung Nr. 15 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 23 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 24 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 52 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan, 1,3-Dichlorpropen, 1,3-Dichlorpropen + 1,2-Dichlorpropan,
1,2-Dibrom-ethan, 2-sec.-Butyl-phenyl-N-methylcarbamat, o-Chlorphenyl-N-methylcarbamat,
3-Isopropyl-5-methylphenyl-N-methylcarbamat,o-Isopropoxyphenyl-N-methylcarbamat,
3,5-Dimethyl-4-methylmercapto-phenyl-N-methylcarbamat,
4-Dimethylamino-3,5-xylyl-N-methylcarbamat, 2-(1,3-Dioxolan-2-yl)-phenyl-N-methylcarbamat,
1-Naphthyl-N-methylcarbamat, 2,3-Dihydro-2,2-dimethyl-benzofuran-7-yl-N-ethylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-1-thiooxamidat,
N-(2-Methyl-chlor-phenyl)-N',N'-dimethylformamidin, Tetrachlorthiophen,
1-(2,6-Difluor-benzoyl)-3-(4-chlor-phenyl)-harnstoff,
O,O-Dimethyl-O-(p-nitrophenyl)-phosphorthioat,
O,O-Diethyl-O-(p-nitrophenyl)-phosphorthioat,
O-Ethyl-O-(p-nitrophenyl)-phenyl-phosphonothioat,
O,O-Dimethyl-O-(3-methyl-4-nitrophenyl)-phosphorthioat,
O,O-Dimethyl-O-(2,4-dichlorphenyl)-phosphorthioat,
O-Ethyl-O-(2,4-dichlorphenyl)-phenyl-phosphonothioat,
O,O-Dimethyl-O-(2,4,5-trichlorphenyl)-phosphorthioat,
O-Ethyl-O-(2,4,5-trichlorphenyl)-ethyl-phosphonothioat,
O,O-Dimethyl-O-(4-brom-2,5-dichlorphenyl)-phosphorthioat,
O,O-Dimethyl-O-(2,5-dichlor-4-jodphenyl)-phosphorthioat,
O,O-Dimethyl-O-(3-methyl-4-methylthiophenyl)-phosphorthioat,
O-Ethyl-O-(3-methyl-4-methylthiophenyl)-isopropyl-phosphoramidat,
O,O-Diethyl-O-[p-methylsulfinyl-phenyl]-phosphorthioat,
O-Ethyl-S-phenyl-ethyl-phosphonodithioat,
O,O-Diethyl-[2-chlor-1-(2,4-dichlorphenyl)-vinyl]-phosphat,
O,O-Dimethyl-[-2-chlor-1-(2,4,5-trichlorphenyl)]-vinyl-phosphat,
O,O-Dimethyl-S-(1'-phenyl)-ethylacetat-phosphordithioat,
Bis-(dimethylamino)-fluorphosphinoxid, Octamethyl-pyrophosphoramid,
O,O,O,O-Tetraethyldithio-pyrophosphat, S-Chlormethyl-O,O-diethyl-phosphordithioat,
O-Ethyl-S,S-dipropyl-phosphordithioat, O,O-Dimethyl-O-2,2-dichlorvinyl-phosphat,
O,O-Dimethyl-1,2-dibrom-2,2-dichlorethylphosphat,
O,O-Dimethyl-2,2,2-trichlor-1-hydroxy-ethylphosphonat,
O,O-Dimethyl-S-[1,2-biscarbethoxy-ethyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,

O,O-Dimethyl-S-(N-methoxyethyl-carbamoyl-methyl)-phosphordithioat,
O,O-Dimethyl-S-(N-formyl-N-methyl-carbamoylmethyl-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diethylcarbamoyl)-vinyl]-phosphat,
O,O-Dimethyl-S-(ethylthio-methyl)-phosphordithioat,
O,O-Dimethyl-S-[(p-chlorphenylthio)-methyl]-phosphordithioat,
O,O-Dimethyl-S-(2-ethylthioethyl)-phosphorthioat,
O,O-Dimethyl-S-(2-ethylthioethyl)-phosphordithioat,
O,O-Dimethyl-S-(2-ethylsulfinyl-ethyl)-phosphorthioat,
O,O-Dimethyl-S-(2-ethylthioethyl)-phosphordithioat,
O,O-Diethyl-S-(2-ethylthioethyl)-phosphorthioat,
O,O-Diethyl-S-(2-ethylsulfinylethyl)-phosphorthioat,
O,O-Diethyl-S-(2-ethylsulfinyl-ethyl)-phosphorthioat,
O,O-Diethyl-thiophosphoryliminophenyl-acetonitril,
O,O-Diethyl-S-(2-chlor-1-phthalimido-ethyl)-phosphordithioat,
O,O-Diethyl-S-[6-chlor-benzoxazolon-(2)-yl(3)]-methyldithiophosphat,
O,O-Dimethyl-S-[2-methoxy-1,3,4-thiadiazol-5-[4H]-onyl-(4)-methyl]-phosphordithioat,
O,O-Diethyl-O-[3,5,6-trichlor-pyridyl-(2)]-phosphorthioat,
O,O-Dimethyl-O-(2-pyrazinyl)-phosphorthioat,
O,O-Diethyl-O-[2-isopropyl-4-methyl-pyrimidinyl(6)]-phosphorthioat,
O,O-Dimethyl-O-[2-(diethylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[(4,6-diamino-1,3,5-triazin-2-yl)-methyl]-phosphordithioat,
O,O-Diethyl-(1-phenyl-1,2,4-triazol-3-yl)-thionophosphat, O,S-Dimethyl-phosphor-amido-thionat,
O,S-Dimethyl-N-acetyl-phosphoramidothioat, $\gamma$-Hexachlorcyclohexan,
1,1-Di-(p-methoxyphenyl)-2,2,2-trichlor-ethan,
6,7,8,9,10,10-Hexachloro-1,5,5a,6,9,9a-hexahydro-6,9-methano-2,4,3-benzodioxa-thiepin-3-oxid, Pyrethrine,
DL-2-Allyl-3-methyl-cyclopenten-(2)-on-(1)-yl-(4)-DL-cis,-trans-chrysanthemat,
5-Benzyl-furyl-(3)-methyl-DL-cis,-trans-chrysanthemat,
3-Phenoxybenzyl($\pm$)-cis,trans-2,2-dimethyl-3-(2,2-dichlorvinyl)-cyclopropancarboxylat,
$\alpha$-Cyano-3-phenoxybenzyl($\pm$)-cis,trans-2,2-dimethyl-3-(2,2-dichlorvinyl)-cyclopropancarboxylat,
(s)-$\alpha$-Cyano-3-phenoxybenzyl-cis(1R,3R)-2,2-dimethyl-3-(2,2-dibromvinyl)--cyclopropancarboxylat, 3,4,5,6-Tetrahydrophthalimidoethyl-DL-cis, trans-chrysanthemat,
2-Methyl-5-(2-propinyl)-3-furylmethyl-chrysanthemat,
($\alpha$-Cyano-3-phenoxybenzyl)-$\alpha$-isopropyl-4-chlorphenylacetat.

Die folgenden Beispiele belegen die biologische Wirkung der neuen Verbindungen. Vergleichsmittel sind die bekannten Wirkstoffe N-(2,6-Difluorbenzoyl)-N'-(3-chlor-4-tert.-butoxycarbonyl-phenyl)-harnstoff, N-(2-Chlorbenzoyl)-N'-(3-chlor-4-tert.-butoxycarbonyl-phenyl)-harnstoff und N-(2-Methylbenzoyl)-N'-(3-chlor-4-tert.-butoxycarbonyl-phenyl)-harnstoff (DE-OS 28 43 852).

Die Numerierung der Wirkstoffe entspricht der der tabellarischen Auflistung.

## Beispiel A

### Zuchtversuch mit Moskito-Larven (Aedes aegpyti)

In 200 ml Leitungswasser, das mit der Wirkstoffaufbereitung aus 10 Gew.-% Wirkstoff und 90 Gew.-% eines Emulgatorgemisches aus 10% ethoxyliertem Rizinusöl, 20% ethoxyliertem Isooctylphenol und 70% Cyclohexanon versetzt wurde, werden 30 bis 40 Aedes-Larven im 4. Stadium gebracht.

Die Versuchstemperatur beträgt 25°C. Beurteilt wird Verpuppung und Schlüpfen der Imagines, wobei eine unbehandelte Kontrolle als Maßstab dient. Während der 12stündigen Versuchsdauer wird einmal mit einem herkömmlichen pulverisierten Fischfutter gefüttert.

In diesem Test zeigen die Wirkstoffe Nr. 1, 2, 3, 5, 6, 9, 10, 13, 16 eine bessere Wirkung als die Vergleichsmittel.

## Beispiel B

### Zuchtversuch mit Baumwollwanzen (Dysdercus intermedius)

50 g Baumwollsaat werden für 24 Stunden in der wäßrigen Aufbereitung von 10 Gew.-% Wirkstoff

und 90 Gew.-% eines Emulgatorgemisches aus 10% ethoxyliertem Rizinusöl, 20% ethoxyliertem Isooctylphenol und 70% Cyclohexanon eingequollen. Die überstehende Flüssigkeit wird abgeschüttet.

Darauf bringt man 20 Wanzen im vorletzten Larvenstadium in 1-l-Gläsern, deren Boden mit feuchtem Sand bedeckt ist. Diese Tiere erhalten 7 Tage lang die vorbehandelten Samen als ausschließliche Nahrung. Anschließend wird unbehandeltes Futter geboten.

Es wird beobachtet, ob die Tiere

a) überleben und
b) sich zu Adulten häuten.

In diesem Test zeigen die Wirkstoffe Nr. 1, 2, 3, 4, 5, 7, 8, 9, 10, 11, 12, 13, 14, 15, 16 eine bessere Wirkung als die Vergleichsmittel.

**Patentansprüche**

1. N-Benzoyl-N'-phenylharnstoffe der Formel

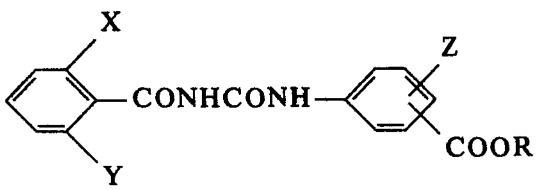

(I)

in der

X für Chlor, Fluor oder Methyl,
Y für Chlor, Fluor oder Wasserstoff,
Z für Brom, Chlor, Fluor oder Wasserstoff und
R für einen primären oder sekundären Alkylrest mit 3 bis 12 Kohlenstoffatomen oder einen gegebenenfalls durch Alkyl substituierten Cycloalkylrest mit 5 bis 12 Kohlenstoffatomen oder den Adamantanylreest stehen.

2. N-Benzoyl-N'-phenylharnstoffe der Formel I

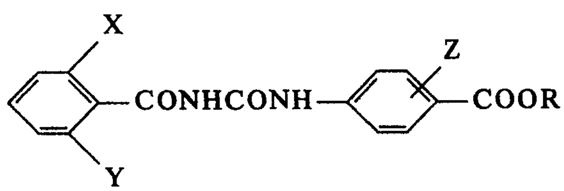

(I)

in der

X für Chlor oder Fluor,
Y für Fluor oder Wasserstoff,
Z für Brom, Chlor, Fluor oder Wasserstoff und
R für einen sekundären Alkylrest mit 4 bis 9 Kohlenstoffatomen stehen.

3. Insektizides Mittel, enthaltend inerte Zusatzstoffe und einen N-Benzoyl-N'-phenylharnstoff der Formel

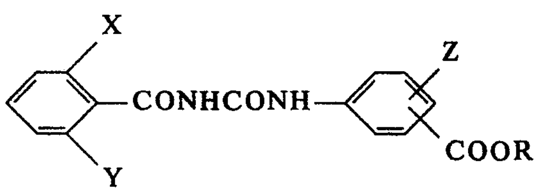

(I)

in der

X für Chlor, Fluor oder Methyl,
Y für Chlor, Fluor oder Wasserstoff,
Z für Brom, Chlor, Fluor oder Wasserstoff und
R für einen primären oder sekundären Alkylrest mit 3 bis 12 Kohlenstoffatomen oder einen gegebenenfalls durch Alkyl substituierten Cycloalkylrest mit 5 bis 12 Kohlenstoffatomen oder den Adamantanylrest stehen,

als Wirkstoff.

4. Insektizides Mittel, enthaltend inerte Zusatzstoffe und einen N-Benzoyl-N'-phenylharnstoff der Formel

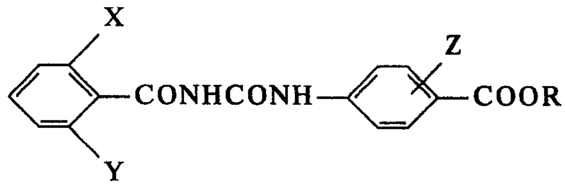

(I)

in der

X für Chlor oder Fluor,
Y für Fluor oder Wasserstoff,
Z für Brom, Chlor, Fluor oder Wasserstoff und
R für einen sekundären Alkylrest mit 4 bis 9 Kohlenstoffatomen stehen,

als Wirkstoff.

5. Verfahren zur Herstellung von N-Benzoyl-N'-phenylharnstoffen der Formel

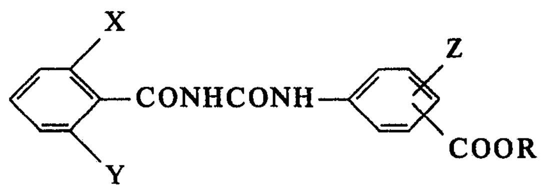

(I)

in der

X für Chlor oder Fluor oder Methyl,
Y für Chlor, Fluor oder Wasserstoff,
Z für Brom, Chlor, Fluor oder Wasserstoff und
R für einen primären oder sekundären Alkylrest mit 3 bis 12 Kohlenstoffatomen oder einen gegebenenfalls durch Alkyl substituierten Cycloalkylrest mit 5 bis 12 Kohlenstoffatomen oder den Adamantanylrest stehen,

dadurch gekennzeichnet, daß man ein Benzoylisocyanat der Formel

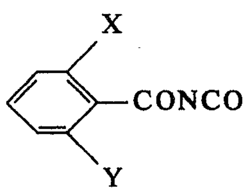

(II)

in der
X und Y die obengenannten Bedeutungen haben,
mit einem Aminobenzoesäureester der Formel

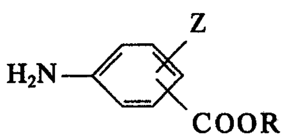

(III)

in der
Z und R die obengenannten Bedeutungen haben,
in Gegenwart eines inerten organischen Lösungsmittels bei einer Temperatur im Bereich zwischen 0 und 80° C umsetzt.

6. Verfahren zur Herstellung von N-Benzoyl-N'-phenylharnstoffen der Formel

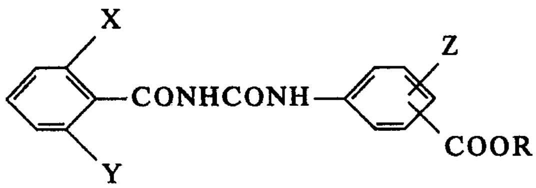

(I)

in der

X für Chlor, Fluor oder Methyl,
Y für Chlor, Fluor oder Wasserstoff,
Z für Brom, Chlor, Fluor oder Wasserstoff und
R für einen primären oder sekundären Alkylrest mit 3 bis 12 Kohlenstoffatomen oder einen gegebenenfalls durch Alkyl substituierten Cycloalkylrest mit 5 bis 12 Kohlenstoffatomen oder den Adamantanylrest stehen,

dadurch gekennzeichnet, daß man ein Amid der Formel

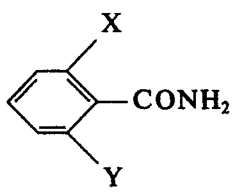

(IV)

in der
X und Y die obengenannten Bedeutungen haben,
mit einem Isocyanat der Formel

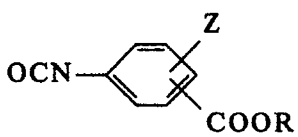

(V)

in der
Z und R die obengenannten Bedeutungen haben,
gegebenenfalls in Gegenwart eines inerten organischen Lösungsmittels bei einer Temperatur zwischen 0 und 140° C umsetzt.

7. Verfahren zur Bekämpfung von Insekten, dadurch gekennzeichnet, daß man eine insektizid wirksame Menge eines N-Benzoyl-N'-phenylharnstoffs der Formel I gemäß Anspruch 1 auf die Insekten und/oder deren Lebensraum einwirken läßt.

8. Verfahren zur Herstellung eines insektiziden Mittels, dadurch gekennzeichnet, daß man mindestens einen N-Benzoyl-N'-phenylharnstoff der Formel I gemäß Anspruch 1 mit oberflächenaktiven Verbindungen und/oder Streckmitteln und/oder Synergisten mischt.

**Claims**

1. An N-benzoyl-N'-phenylurea of the formula

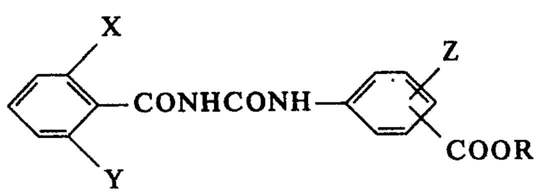

(I)

where X is chlorine, fluorine or methyl, Y is chlorine, fluorine or hydrogen, Z is bromine, chlorine, fluorine or hydrogen, and R is primary or secondary alkyl of 3 to 12 carbon atoms, unsubstituted or alkyl-substituted cycloalkyl of 5 to 12 carbon atoms or adamantanyl.

2. An N-benzyl-N′-phenylurea of the formula

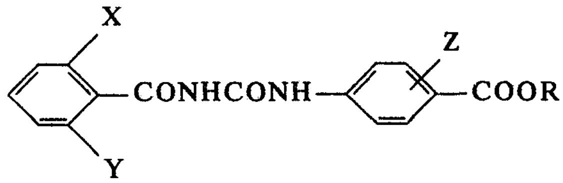

(I)

where X is chlorine or fluorine, Y is fluorine or hydrogen, Z is bromine, chlorine, fluorine or hydrogen, and R is secondary alkyl of 4 to 9 carbon atoms.

3. An insecticidal agent containing inert additives and, as active ingredient, an N-benzoyl-N′-phenylurea of the formula

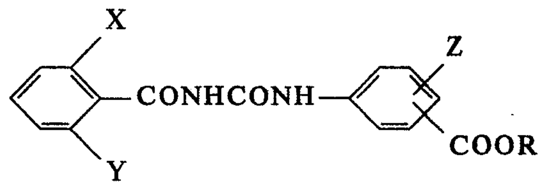

(I)

where X is chlorine, fluorine or methyl, Y is chlorine, fluorine or hydrogen, Z is bromine, chlorine, fluorine or hydrogen, and R is primary or secondary alkyl of 3 to 12 carbon atoms, unsubstituted or alkyl-substituted cycloalkyl of to 12 carbon atoms or adamantanyl.

4. An insecticidal agent containing inert additives and as active ingredient, an N-benzoyl-N′-phenylurea of the formula

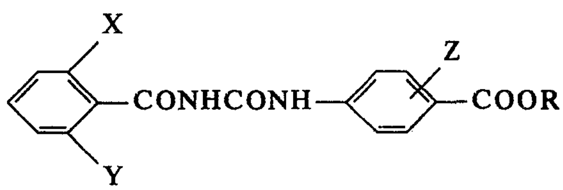

(I)

where X is chlorine or fluorine, Y is fluorine or hydrogen, Z is bromine, chlorine, fluorine or hydrogen, and R is secondary alkyl of 4 to 9 carbon atoms.

5. A process for the preparation of an N-benzoyl-N′-phenyl-urea of the formula

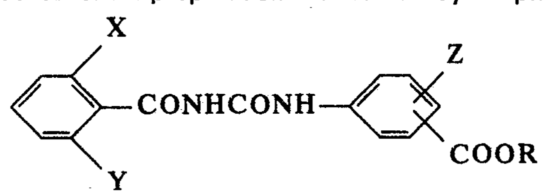

(I)

where X is chlorine, fluorine or methyl, Y is chlorine, fluorine or hydrogen, Z is bromine, chlorine, fluorine or hydrogen, and R is primary or secondary alkyl of 3 to 12 carbon atoms, unsubstituted or alkyl-substituted cycloalkyl of 5 to 12 carbon atoms or adamantanyl,
wherein a benzoyl isocyanate of the formula

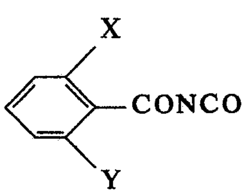

(II)

where X and Y have the above meanings, is reacted with an aminobenzoic acid ester of the formula

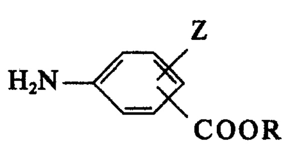

(III)

where Z and R have the above meanings, in the presence of an inert organic solvent at from 0 to 80° C.

6. A process for the preparation of an N-benzoyl-N'-phenylurea of the formula

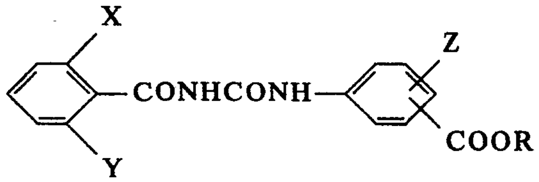

(I)

where X is chlorine, fluorine or methyl, Y is chlorine, fluorine or hydrogen, Z is bromine, chlorine, fluorine or hydrogen, and R is primary or secondary alkyl of 3 to 12 carbon atoms, unsubstituted or alkyl-substituted cycloalkyl of 5 to 12 carbon atoms or adamantanyl,
wherein an amide of the formula

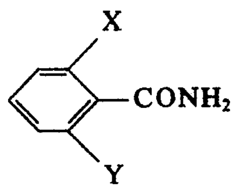

(IV)

where X and Y have the above meanings, is reacted with an isocyanate of the formula

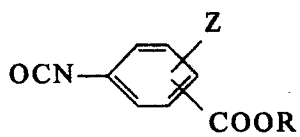

(V)

where Z and R have the above meanings, in the presence or absence of an inert organic solvent, at from 0 to 140°C.

7. A process for combating insects, wherein an insecticidally effective amount of an N-benzoyl-N'-phenylurea of the formula I as claimed in claim 1 is allowed to act on the insects and/or their habitat.

8. A process for the production of an insecticidal agent, wherein at least one N-benzoyl-N'-phenylurea of the formula I as claimed in claim 1 is mixed with surface-active compounds and/or extenders and/or synergists.

**Revendications**

1. N-benzoyl-N'-phénylurée de formule

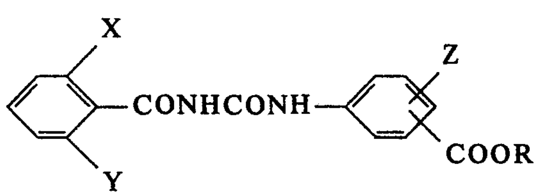

(I)

dans laquelle

X représente chlore, fluor ou méthyle,
Y représente chlore, fluor ou hydrogène,
Z représente brome, fluor ou hydrogène et
R représente un reste alkyle primaire ou secondaire, ayant 3 à 12 atomes de carbone ou un reste cycloalkyle ayant 5 à 12 atomes de carbone et éventuellement substitué par alkyle ou le reste adamantanyle.

2. N-benzoyl-N'-phénylurée de formule I

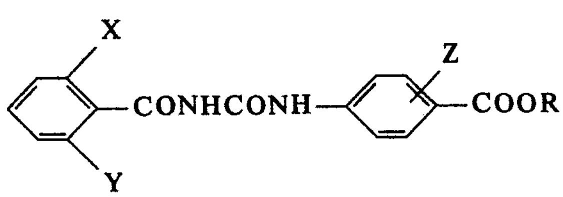

(I)

dans laquelle

X représente chlore, fluor ou méthyle,
Y représente chlore, fluor ou hydrogène,
Z représente brome, chlore, fluor ou hydrogène et
R représente un reste alkyle secondaire ayant 4 à 9 atomes de carbone.

3. Agent insecticide, contenant des additifs inertes et, comme principe actif, une N-benzoyl-N'-phénylurée de formule

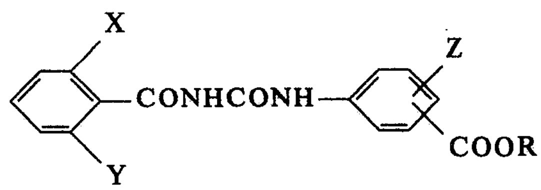

(I)

dans laquelle

X représente chlore, fluor ou méthyle,
Y représente chlore, fluor ou hydrogène,
Z représente brome, fluor ou hydrogène et
R représente un reste alkyle primaire ou secondaire, ayant 3 à 12 atomes de carbone ou un reste cycloalkyle ayant 5 à 12 atomes de carbone et éventuellement substitué par alkyle ou le reste adamantanyle.

4. Agent isecticide, contenant des additifs inertes et, comme principe actif, une N-benzoyl-N'-phénylurée de formule

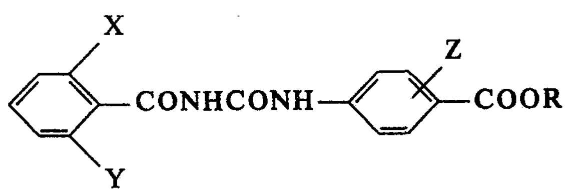

(I)

dans laquelle

X représente chlore, fluor ou méthyle,
Y représente chlore, fluor ou hydrogène,
Z représente brome, chlore, fluor ou hydrogène et
R représente un reste alkyle secondaire ayant 4 à 9 atomes de carbone.

5. Procédé de préparation de N-benzoyl-N'-phénylurée de formule

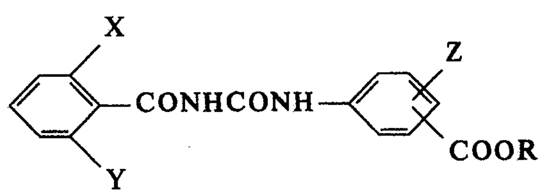

(I)

dans laquelle

X représente chlore, fluor ou méthyle,
Y représente chlore, fluor ou hydrogène,
Z représente brome, chlore, fluor ou hydrogène et
R représente un reste alkyle primaire ou secondaire, ayant 3 à 12 atomes de carbone ou un reste cycloalkyle ayant 5 à 12 atomes de carbone et éventuellement substitué par alkyle ou le reste adamantanyle,

caractérisé par le fait qu'on fait réagir un isocyanate de benzoyle de formule

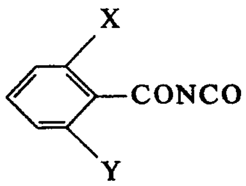

(II)

dans laquelle
X et Y ont les significations sus-mentionnées avec un ester d'acide aminobenzoïque de formule

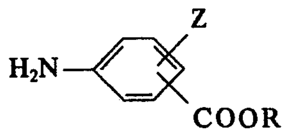

(III)

dans laquelle
Z et R ont les significations susmentionnées, en présence d'un solvant organique inerte, à une température comprise entre 0 et 80° C.

6. Pocédé de préparation de N-benzoyl-N'-phénylurée de formule

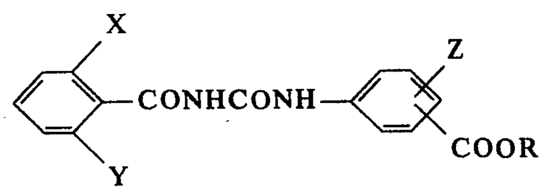

(I)

dans laquelle

X représente chlore, fluor ou méthyle,
Y représente chlore, fluor ou hydrogène
Z représente brome, chlore, fluor ou hydrogène et
R représente un reste alkyle primaire ou secondaire, ayant 3 à 12 atomes de carbone ou un reste cycloalkyle ayant 5 à 12 atomes de carbone et éventuellement substitué par alkyle ou le reste adamantanyle,

caractérisé par le fait qu'on fait réagir un amide de formule

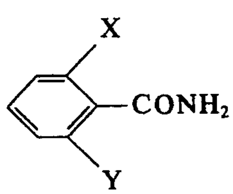

(IV)

dans laquelle
X et Y ont les significations sus-indiquées avec un isocyanate de formule

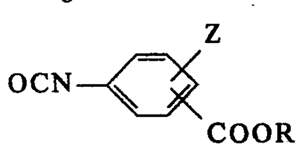

(V)

dans laquelle
Z et R ont les significations sus-mentionnées, éventuellement en présence d'un solvant organique inerte, à une température comprise entre 0 et 140° C.

7. Procédé de lutte contre les insectes, caractérisé par le fait qu'on fait agir sur les insectes et/ou leur biotope une quantité efficace au point de de vue insecticide d'une N-benzoyl-N'-phénylurée de formule I selon la revendication 1.

8. Procédé de préparation d'un agent insecticide, caractérisé par le fait qu'on mélange au moins une N-benzoyl-N'-phénylurée de formule I selon la revendication 1 avec des composés tensioactifs et/ou extendeurs et/ou synergistes.